# EUROPÄISCHE PATENTSCHRIFT

(11) **EP 0 701 824 B1**
(45) Veröffentlichungstag und Bekanntmachung des Hinweises auf die Patenterteilung: **19.07.2000**
(21) Anmeldenummer: 95114027.6
(22) Anmeldetag: 07.09.1995
(51) Int. Cl.: A61L 24/00, A61L 27/00

(54) **Verfahren zur Herstellung von wirkstoffhaltigen Knochenzementen**
Method of preparing bone cements containing active agents
Procédé pour la préparation de ciments osseaux contenant des agents actifs

(30) Priorität: 17.09.1994 DE 4433201
(43) Veröffentlichungstag der Anmeldung: 20.03.1996
(73) Patentinhaber: MERCK PATENT GmbH, 64293 Darmstadt (DE)
(72) Erfinder: Franz, Hans-Werner, D-64807 Dieburg (DE); Nies, Berthold, Dr., D-64407 Fränkisch-Crumbach (DE)

(56) Entgegenhaltungen:
- EP-A- 0 202 445
- EP-A- 0 301 759
- DD-A- 207 655
- DE-A- 2 022 117
- LANGENBECKS ARCHIV FUR CHIRURGIE, Bd. 371, Nr. 2, 1987, Seiten 123-136, XP000671040 LANGENDORFF: "Cytostaticahaltiger Knochenzement: Neue Aspekte in der Behandlung"

## Beschreibung

Die Erfindung betrifft ein Verfahren zur Herstellung von wirkstoffhaltigen Knochenzementen sowie daraus hergestellter Knochenersatzmaterialien oder implantierbarer Pharmakadepots.

Knochenzemente, Knochenersatzmaterialien und implantierbare Pharmakadepots, die auf Acrylatkunststoffen basieren, sind seit langem bekannt. Polymermaterialien auf Basis von Acryl- und/oder Methacrylsäureestern haben sich hier aufgrund ihrer Biokompatibilität, ihrer vorzüglichen Festigkeitseigenschaften, ihrer günstigen Eigenschaften bei der Freisetzung eingelagerter pharmazeutischer Wirkstoffe und nicht zuletzt aufgrund ihrer anwendungsgerechten Verarbeitbarkeit bewährt.

Gängige Knochenzemente setzen sich aus etwa 50 bis 75 Gew.% einer Feststoffkomponente, die aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen besteht, und aus etwa 25 bis 50 Gew.% einer flüssigen Komponente, die aus einem Acryl- und/oder Methacrylsäureestermonomeren sowie gegebenenfalls weiteren Zusätzen wie Polymerisationsbeschleunigern und Stabilisatoren besteht, zusammen. Zum Gebrauch werden Feststoffkomponente und flüssige Komponente zu einer flüssigen bis halbfesten Paste vermengt, diese gegebenenfalls in eine gewünschte Form gebracht oder zur Einzementierung einer Prothese an dem Implantationsort appliziert. Die Aushärtung der Masse erfolgt durch die mit dem Vermischen der Komponenten induzierte Polymerisationsreaktion.

Sehr gebräuchlich ist zum Beispiel ein Knochenzement, der in einer Normalpackung 2 Beutel mit je etwa 40 g Polymerpulver und 2 Ampullen mit je 20 ml Monomerflüssigkeit enthält. Das Pulver ist ein feines Perlpolymerisat aus Methacrylsäuremethylester mit einem Copolymeranteil von Acrylsäuremethylester. Als Katalysator sind dem Pulver etwa 0,5 % Dibenzoylperoxid zugesetzt. Zur Kennzeichnung des Materials sind bei der Herstellung geringe Mengen von Chlorophyll miteinpolymerisiert. Das Pulver enthält zusätzlich ein übliches Röntgenkontrastmittel wie zum Beispiel Zirkondioxid. Die zugehörige Flüssigkeit besteht aus monomerem Methacrylsäuremethylester, dem als Polymerisationsbeschleuniger etwa 0,7 % Dimethyl-p-toluidin sowie als Stabilisator geringe Mengen von Hydrochinon zugesetzt sind. Auch diese Flüssigkeit ist in der Regel zur Kennzeichnung mit einer geringen Menge von Chlorophyll eingefärbt. Das in Polyethylenbeutel abgepackte Pulver ist mit Ethylenoxid sterilisiert. Die Flüssigkeit ist sterilfiltriert und in Glasampullen abgefüllt.

Beim Zusammenmischen von 2 Gewichtsteilen Pulver mit einem Gewichtsteil Flüssigkeit reagiert das Dibenzoylperoxid mit dem Dimethyl-p-toluidin in der Flüssigkeit, wodurch die radikalische Polymerisation angeregt wird. Die Mischung ist so abgestimmt, daß sie schon nach etwa einer Minute als teigige Paste verwendet werden kann. Diese Paste bleibt für mehrere Minuten knetbar und beginnt dann unter Wärmeentwicklung auszuhärten. Nach etwa 5 bis 10 Minuten ist die Polymerisation im wesentlichen abgeschlossen. Während der Polymerisationsphase, solange die Paste noch formbar ist, kann diese in jede gewünschte Form gebracht werden, also zum Beispiel zum Ausfüllen von Knochenhöhlen oder zum Einzementieren von Prothesen direkt in den Körper gebracht oder zur Herstellung von Formkörpern verwendet werden, die extrakorporal aushärten und danach an beliebigen Körperstellen eingesetzt werden können.

Bei zahlreichen Indikationen ist es wünschenswert, daß der Knochenzement pharmazeutische Wirkstoffe enthält. So können cytostatikahaltige Knochenzemente bei der Sanierung von Knochendefekten nach Entfernung von Knochentumoren eingesetzt werden. Bei der Einzementierung von Prothesen und bei der Osteosynthese sind Knochenzemente, die Antibiotika, Antiseptika sowie gegebenenfalls knochenwachstumsfördernde Substanzen enthalten, vorteilhaft. Formkörper aus wirkstoffhaltigen Knochenzementen können in Weichteilgewebe als lokale Wirkstoffdepots mit verzögerter Wirkstofffreisetzung implantiert werden.

In EP 0 202 445 A1 ist beispielsweise ein derartiger cytostatikahaltiger Knochenzement sowie ein daraus hergestelltes Pharmakadepot mit besonders günstigen Freisetzungseigenschaften beschrieben. Aus dieser Druckschrift geht hervor, daß der jeweilige Wirkstoff dem Basismaterial des Knochenzementes, also dem Präpolymeren und/oder dem Monomeren als feinteiliges Pulver, zugemischt wird, so daß es dann in dem entstehenden Polymerisat homogen verteilt vorliegt.

In der Praxis hat sich hier jedoch gezeigt, daß eine Bereitstellung der Knochenzementkomponenten in vorkonfektionierter Form, bei der entweder eine der Komponenten, vorzugsweise das Polymerisat, bereits den pharmazeutischen Wirkstoff enthält oder bei der der Wirkstoff separat gepackt ist, um bei der Anmischung zugegeben zu werden, nicht den Anforderungen, die an ein für die Implantation in den Körper vorgesehenes medizinisches Produkt gestellt werden müssen, entspricht. Bei einem derartigen wirkstoffhaltigen Knochenzement bzw. seinen Komponenten ist es nämlich nicht möglich, die erforderliche und bei entsprechenden wirkstofffreien Produkten problemlose Endsterilisation nach üblichen Methoden wie Bestrahlen mit γ-Strahlung oder Begasung mit Ethylenoxid durchzuführen. Zahlreiche der hier in Frage kommenden pharmazeutischen Wirkstoffe sind empfindlich gegenüber Einwirkung von γ-Strahlung oder Ethylenoxid. Dies trifft in besonderem Maße auch auf Cytostatika wie etwa dem hier bevorzugt eingesetzten Wirkstoff Methotrexat zu. Eine Sterilfiltration der flüssigen Monomerkomponente, der feinpulvriger Wirkstoff zugesetzt ist, führt letztendlich zur Abtrennung des im Monomer im wesentlichen unlöslichen Wirkstoffes. Die Bereitstellung aller dieser drei Knochenzementkomponenten würde demnach die Verfügbarkeit des pharmazeutischen Wirkstoffes aus einer sterilen Produktionslinie erfordern. Die Produktion eines pharmazeutischen Wirkstoffes unter durchgängig sterilen Bedingungen ist aus naheliegenden Gründen äußerst aufwendig.

Es bestand daher die Aufgabe ein Verfahren zu entwickeln, mit dem wirkstoffhaltige Knochenzemente bzw. deren Vor- sowie auch ihre Folgeprodukte auf einfache Weise in steriler Form bereitgestellt werden können.

Es wurde nun gefunden, daß ein Knochenzement, der sich zusammensetzt aus einer polymeren Feststoffkomponente, einer flüssigen Monomerkomponente und einer Lösung eines pharmazeutischen Wirkstoffes in einem organischen Lösungsmittel, dessen Anteil 50 Gew.%, bezogen auf die flüssige Komponente, nicht übersteigt, praktisch keine Veränderung hinsichtlich Verarbeitbarkeit, Aushärtungsverhalten und mechanischer Festigkeit zeigt. Bei dieser Zusammensetzung und Vorgehensweise ist es möglich, die Wirkstofflösung vor der Vereinigung mit der Monomerkomponente oder der Feststoffkomponente einer Sterilfiltration zu unterziehen, so daß in einfacher Weise alle diese drei Komponenten des Knochenzementes in steriler Form zur Verfügung gestellt werden können.

Gegenstand der Erfindung ist somit ein Verfahren zur Herstellung von wirkstoffhaltigen Knochenzementen sowie daraus hergestellter Knochenersatzmaterialien oder implantierbarer Pharmakadepots, wobei sich der Knochenzement aus etwa 50 bis 75 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, und aus etwa 25 bis 50 Gew.% einer flüssigen Komponente, bestehend aus einem Acryl- und/oder Methacrylsäureestermonomeren sowie gegebenenfalls weiteren Zusätzen wie Polymerisationsbeschleunigern und Stabilisatoren, zusammensetzt, die zu einer flüssigen bis halbfesten Paste vermengt, gegebenenfalls in eine gewünschte Form gebracht und dann ausgehärtet werden, das dadurch gekennzeichnet ist, daß man den Wirkstoff in einem organischen Lösungsmittel, dessen Anteil 50 Gew.%, bezogen auf die flüssige Komponente, nicht übersteigt, auflöst und diese Lösung mit der flüssigen Komponente oder der Feststoffkomponente vermischt.

Bei dem erfindungsgemäßen Verfahren lassen sich alle üblichen Knochenzemente auf Acrylat-/Methacrylat-Basis bzw. die hierfür gebräuchlichen Ausgangsprodukte verwenden. Knochenzemente dieser Art sind im Handel erhältlich. Ihre Zusammensetzung und die Art ihrer Verarbeitung sind dem Fachmann geläufig.

Zur Herstellung eines wirkstoffhaltigen Knochenzementes ist es erfindungsgemäß vorgesehen den pharmazeutischen Wirkstoff zunächst in einem organischen Lösungsmittel aufzulösen und dann diese Wirkstofflösung mit der flüssigen Monomerkomponente oder der feinteiligen polymeren Feststoffkomponente des Knochenzementes zu vermischen. Die Wirkstofflösung ist ohne weiteres durch Sterilfiltration sterilisierbar, so daß hiermit alle Komponenten des wirkstoffhaltigen Knochenzementes, also auch die Wirkstofflösung, sterilisiert, in steriler Form bereitgehalten und unter Sterilbedingungen zum anwendungsfertigen Knochenzement verarbeitet werden können.

Für die Bereitung der Wirkstofflösung sind im wesentlichen alle üblichen organischen Lösungsmittel geeignet. Zweckmäßigerweise werden solche Lösungsmittel ausgewählt, in denen einerseits das vorgesehene Pharmazeutikum gut löslich ist, so daß eine möglichst hochkonzentrierte Lösung hergestellt werden kann, und mit dem sich andererseits die flüssige Monomerkomponente gut zu einer homogenen Phase mischt. Die Menge an Lösungsmittel wird so gewählt, daß sie 50 Gew.%, bezogen auf die flüssige Monomerkomponente, nicht übersteigt. Hierdurch ist gewährleistet, daß sich bei der Mischung der Komponenten zum gebrauchsfertigen Knochenzement die Verarbeitungseigenschaften, die Aushärtungscharakteristik und die mechanische Festigkeit des ausgehärteten Knochenzementes nicht verändern. Vorzugsweise wird eine solche Menge an Lösungsmitteln gewählt, daß deren Anteil 5 bis 25 Gew.% und insbesondere 10 bis 15 Gew.%, bezogen auf die flüssige Komponente, beträgt.

Die zum Einsatz gelangende Menge an pharmazeutischem Wirkstoff ist abhängig von dessen spezifischer Wirksamkeit, der medizinischen Indikation und vom jeweiligen Anforderungsprofil des Knochenzementes bzw. des daraus herzustellenden Knochenersatzmaterials oder Pharmakadepots. In aller Regel ist ein Anteil an pharmazeutischem Wirkstoff von 0,1 bis 5 Gew.%, bezogen auf die Gesamtmenge an Knochenzement, ausreichend; in Einzelfällen, insbesondere bei der Herstellung von implantierbaren Pharmakadepots, kann der Wirkstoffanteil auch höher, etwa bis zu 40 Gew.% liegen.

Erfindungsgemäß können alle pharmazeutischen Wirkstoffe in dieser Weise in Knochenzemente eingearbeitet werden, die zum einen von ihrem Wirkungsprofil her in Knochenzementen, in Knochenersatzwerkstoffen sowie in implantierbaren Pharmakadepots sinnvoll sind und die zum anderen gegenüber den Bestandteilen von Knochenzementen sowie den bei der Aushärtung entstehenden Temperaturen ausreichend stabil sind. Als Wirkstoffe kommen vorzugsweise Cytostatika wie Methotrexat, Cisplatin, Cyclophosphamid, Fluoruracil, Doxorubicin etc., Antibiotika wie Gentamicin, Clindamycin, Vancomycin, Teicoplanin etc., weiterhin Antiseptika sowie knochenwachstumsfördernde Substanzen in Betracht. Das erfindungsgemäße Verfahren eignet sich im besonderen Maße zur Herstellung von Knochenzementen, die Cytostatika enthalten und für die nach bisherigem Verfahren eine Endsterilisation nicht möglich ist. Als besonders geeignet und vorteilhaft hat es sich für das Cytostatikum Methotrexat erwiesen.

Überraschend hat sich hier gezeigt, daß das Freisetzungsverhalten von methotrexathaltigem Knochenzement, der nach dem erfindungsgemäßen Verfahren hergestellt worden ist, erheblich besser ist, als wenn Methotrexat wie bisher lediglich in Form von feinteiligem Feststoff den Knochenzementkomponenten zugemischt wird. Die Freisetzungscharakteristik entspricht etwa der eines methotrexathaltigen Knochenzementes, dem zur Verbesserung der Wirkstofffreisetzung Zusätze wie Aminosäuren, insbesondere wie in EP 0 202 445 A1 beschrieben, mit besonders feinteiligen Partikeln zugefügt worden sind. Bei nach dem erfindungsgemäßen Verfahren hergestellten Knochenzementen kann daher auf derartige Zusätze verzichtet werden, ohne Einbußen in der Freisetzungscharakteristik hinnehmen zu müssen.

Als für Methotrexat gut geeignete Lösungsmittel haben sich 2-Pyrrolidon, N-Methylpyrrolidon, Dimethylsulfoxid (DMSO), Tetrahydrofuran, Dioxan, Ethylenglycol, Propandiol oder Kombinationen hiervon herausgestellt. Besonders bevorzugt sind 2-Pyrrolidon, N-Methylpyrrolidon sowie ein Gemisch aus DMSO und Propandiol im Verhältnis 1:1.

Methotrexat kann in Form des Dinatriumsalzes oder auch in Form der freien Säure eingesetzt werden. Die Löslichkeit des Wirkstoffes in den genannten Lösungsmitteln ist so, daß für den für Methotrexat üblichen Bereich der Anwendungsmenge, nämlich 0,1 bis 4 Gew.%, bezogen auf die Gesamtmenge an Knochenzement, die Lösungsmittelmenge ohne weiteres auf zum Beispiel ein Zehntel der Monomermenge gewählt werden kann.

Bei Zugabe und Vermischung der Methotrexatlösung mit dem flüssigen Monomer erfolgt eine Ausfällung des Wirkstoffes aus dem Flüssigkeitsgemisch in Form eines feindispersen Feststoffes. Es wird angenommen, daß das Vorliegen des Wirkstoffes im Knochenzement in dieser feindispersen Form verantwortlich ist für die gegenüber üblicher Zumischung verbesserte Freisetzungscharakteristik.

Selbstverständlich kann das Freisetzungsverhalten des Wirkstoffes durch die bekannten und üblichen Zusätze noch beeinflußt und gegebenenfalls weiter verbessert werden. Als derartige Zusätze kommen Aminosäuren wie Arginin sowie Hydroxylapatit oder Natriumhydrogencarbonat, möglichst in feinteiliger Form mit Partikelgrößen unter 100 µm, in Frage. Durch solche Zusätze läßt sich insbesondere die Anfangskonzentration der Wirkstofffreisetzung regeln.

Die Feststoffkomponente, die üblicherweise als Peripolymerisat von Methylmethacrylat-Methylacrylat-Copolymer mit Partikelgrößen zwischen 5 und 250 um vorliegt, enthält einen Polymerisationskatalysator wie etwa Dibenzoylperoxid. Weiterhin kann sie Röntgenkontrastmittel wie zum Beispiel Zirkondioxid, Farbstoffe zur Kennzeichnung wie etwa Chlorophyll sowie Füllstoffe und gegebenenfalls weitere Zusätze enthalten. Gängige Füllstoffe sind beispielsweise osteoinduktiv bzw. osteokonduktiv wirkende Calciumphosphate wie insbesondere Hydroxylapatit und Tricalciumphosphat. Der Anteil aller dieser Zusätze kann in einem weiteren Bereich variieren und ist abhängig vom jeweiligen Anforderungsprofil des Knochenzementes bzw. der entsprechenden Folgeprodukte. Er übersteigt in der Regel kaum 30 Gew.%, bezogen auf die Feststoffkomponente. Die flüssige Monomerkomponente Methylmethacrylat enthält in aller Regel einen Polymerisationsbeschleuniger wie Dimethyl-p-toluidin sowie Hydrochinon als Stabilisator in den hierfür üblichen Mengen. Weiterhin können Farbstoffe und sonstige zweckmäßige Zusätze vorhanden sein. Die Feststoffkomponente läßt sich ohne weiteres mit γ-Strahlung oder mit Ethylenoxid sterilisieren; die flüssige Komponente und die Wirkstofflösung können jeweils einer Sterilfiltration unterzogen werden. Beide Komponenten können getrennt und steril in entsprechende Behältnisse abgefüllt werden.

Der wirkstoffhaltige Knochenzement wird zweckmäßigerweise in Form eines Sets bereitgestellt, das sich aus getrennten Packungen der drei Hauptkomponenten zusammensetzt. Komponente (a) beinhaltet die Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, deren Anteil etwa 50 bis 75 Gew.% des Knochenzementes beträgt. Komponente (b) beinhaltet die flüssige Komponente, bestehend aus einem Acryl- und/oder Methacrylsäureestermonomeren sowie gegebenenfalls weiteren Zusätzen wie Polymerisationsbeschleunigern und Stabilisatoren, deren Anteil etwa 25 bis 50 Gew.% des Knochenzementes beträgt. Komponente (c) beinhaltet die Lösung des Wirkstoffes in einem organischen Lösungsmittel, dessen Anteil 50 Gew.%, bezogen auf die flüssige Komponente, nicht übersteigt.

Vorzugsweise sind die Mengen der Komponenten so aufeinander abgestimmt, daß die gesamten drei Packungsinhalte miteinander vereinigt werden. Die Mengenabstimmung erfolgt nach Maßgabe des vorgesehenen Anwendungszweckes und je nach dem, ob ein niedrigviskoser, ein mittelviskoser oder ein hochviskoser Zement erwünscht ist. Die Feststoffkomponente ist dabei einer Endsterilisation mit mittels Strahlung oder Ethylenoxid, die flüssige Monomerkomponente und die Wirkstofflösung sind jeweils einer Sterilfiltration unterzogen worden und steril in geeignete Packmittel abgefüllt.

Zweckmäßig ist die Ergänzung dieses Sets mit einer Vorrichtung zum Anmischen und/oder Ausbringen des Knochenzementes. Entsprechende Vorrichtungen sind bekannt und gebräuchlich. Vorzugsweise ermöglichen entsprechende Vorrichtungen das Anmischen des Knochenzementes unter Vakuum sowie das kombinierte Ausbringen des Zementes mittels einer Knochenzementspritze.

Die Herstellung des gebrauchsfertigen wirkstoffhaltigen Knochenzementes und seine weitere Verarbeitung erfolgen in völliger Analogie zu bisherigen Knochenzementsystemen, nur daß zunächst die Wirkstofflösung und die flüssige Monomerkomponente zusammengebracht und miteinander vermischt werden und dann erst zu diesem Gemisch das Polymerpulver zugegeben wird. Gleichermaßen kann man auch zunächst das Polymerpulver mit der Wirkstofflösung vermischen und danach das Monomer zugeben. Nach inniger Durchmischung der Komponenten setzt durch den enthaltenen Katalysator die Polymerisation ein; für den Zeitraum einiger Minuten bleibt die Masse flüssig bis plastisch verformbar; danach liegt das ausgehärtete Endprodukt vor.

Der wirkstoffhaltige Knochenzement kann in üblicher Weise während des flüssigen bzw. plastischen Stadiums für die Implantation von Knochenprothesen verwendet werden. Der Chirurg kann auch die Masse zu Formkörpern beliebiger Form und Größe verarbeiten und diese nach der Aushärtung als lokale Wirkstoffdepots in die zu behandelnden Körperbereiche implantieren. Derartige implantierbare Pharmakadepots können auch bereits vorgefertigt angeboten werden.

### Beispiel

125 g Methotrexat-Säure werden in 1 l 2-Pyrrolidon gelöst und über ein 0,2-µm-Teflonfilter sterilfiltriert, zu jeweils 1 ml in braune Injektionsflaschen steril abgefüllt und verschlossen.

Mit einer sterilen Spritze wird die Methotrexat-Lösung aus der Flasche entnommen und mit 10 ml Methylmethacrylat gemischt. Methotrexat fällt dabei feindispers aus.

Zu der Suspension wird Knochenzementpulver der Zusammensetzung 15,5 g PMMA/PMA-Copolymer (94/6), 3 g Hydroxylapatitpulver (2-5 µm) und 2 g Zirkondioxidpulver gegeben.

Monomer und Polymer enthalten das übliche Startersystem aus Dimethyl-para-Toluidin und Di-Benzoylperoxid.

Nach gründlicher Mischung ist der Zement einsatzbereit.

## Patentansprüche

1. Verfahren zur Herstellung von wirkstoffhaltigen Knochenzementen sowie daraus hergestellter Knochenersatzmaterialien oder implantierbarer Pharmakadepots, wobei sich der Knochenzement aus etwa 50 bis 75 Gew.% einer Feststoffkomponente, bestehend aus einem feinteiligen Polymerisat von Acryl- und/oder Methacrylsäureestern sowie gegebenenfalls weiteren Zusätzen wie Polymerisationskatalysatoren, Röntgenkontrastmitteln, Füllstoffen und Farbstoffen, und aus etwa 25 bis 50 Gew.% einer flüssigen Komponente, bestehend aus einem Acryl- und/oder Methacrylsäureestermonomeren sowie gegebenenfalls weiteren Zusätzen wie Polymerisationsbeschleunigern und Stabilisatoren, zusammensetzt, die zu einer flüssigen bis halbfesten Paste vermengt, gegebenenfalls in eine gewünschte Form gebracht und dann ausgehärtet werden, dadurch gekennzeichnet, daß man den Wirkstoff in einem organischen Lösungsmittel, dessen Anteil 50 Gew.%, bezogen auf die flüssige Komponente, nicht übersteigt, auflöst und diese Lösung mit der flüssigen Komponente oder mit der Feststoffkomponente vermischt.

2. Verfahren nach Anspruch 1, dadurch gekennzeichnet, daß der Anteil an Lösungsmittel 5 bis 25 Gew.%, vorzugsweise 10 bis 15 Gew.%, bezogen auf die flüssige Komponente, beträgt.

3. Verfahren nach den Ansprüchen 1 oder 2, dadurch gekennzeichnet, daß die Feststoffkomponente einer Endsterilisation mittels Strahlung und/oder Ethylenoxid unterzogen und die flüssige Komponente und die Wirkstofflösung sterilfiltriert werden.

4. Verfahren nach den Ansprüchen 1 bis 3, dadurch gekennzeichnet, daß die Wirkstoffe Cytostatika, Antibiotika, Antiseptika oder knochenwachstumsfördernde Substanzen sind.

5. Verfahren nach Anspruch 4, dadurch gekennzeichnet, daß als Wirkstoff Methotrexat verwendet wird.

6. Verfahren nach den Ansprüchen 1 bis 5, dadurch gekennzeichnet, daß als Lösungsmittel 2-Pyrrolidon, N-Methylpyrrolidon, DMSO, Tetrahydrofuran, Dioxan, Ethylenglycol, Propandiol oder Kombinationen hiervon verwendet werden.

7. Set zur Herstellung eines wirkstoffhaltigen Knochenzementes, zusammengesetzt aus getrennten Packungen von
(a) einer Feststoffkomponente, bestehend aus einem feinteiligen Polymerisat von Acryl- und/oder Methacrylsäureestern sowie gegebenenfalls weiteren Zusätze wie Polymerisationskatalysatoren, Röntgenkontrastmitteln, Füllstoffen und Farbstoffen, deren Anteil etwa 50 bis 75 Gew.% des Knochenzementes beträgt,
(b) einer flüssigen Komponente, bestehend aus einem Acryl- und/ oder Methacrylsäureestermonomeren sowie gegebenenfalls weiteren Zusätzen wie Polymerisationsbeschleunigern und Stabilisatoren, deren Anteil etwa 25 bis 50 Gew.% des Knochenzementes beträgt, und
(c) einer Lösung eines Wirkstoffes in einem organischen Lösungsmittel, dessen Anteil 50 Gew.%, bezogen auf die flüssige Komponente, nicht übersteigt,
sowie gegebenenfalls einer Vorrichtung zum Anmischen und/oder Ausbringen des Knochenzementes.

8. Set nach Anspruch 7, dadurch gekennzeichnet, daß die Packungseinheit (a) einer Endsterilisation mittels Strahlung und/oder Ethylenoxid und der Inhalt der Packungseinheiten (b) und (c) einer Sterilfiltration unterzogen worden sind.

9. Set nach Anspruch 8, dadurch gekennzeichnet, daß in Packungseinheit (c) als Wirkstoff Methotrexat enthalten ist.

## Claims

1. Process for the preparation of bone cements comprising active compounds and bone replacement materials or implantable drug depots produced therefrom, the bone cement being composed of about 50 to 75% by weight of a solid component comprising a finely divided polymer of acrylic and/or methacrylic acid esters and if appropriate other additives, such as polymerization catalysts, X-ray contrast media, fillers and dyestuffs, and about 25 to 50% by weight of a liquid component comprising an acrylic and/or methacrylic acid ester monomer and if appropriate other additives, such as polymerization accelerators and stabilizers, which are mixed to form a liquid to semi-solid paste, brought into a desired shape, if appropriate, and then hardened, characterized in that the active compound is dissolved in an organic solvent, the content of which does not exceed 50% by weight, based on the liquid component, and this solution is mixed with the liquid component or the solid component.

2. Process according to Claim 1, characterized in that the content of solvent is 5 to 25% by weight, preferably 10 to 15% by weight, based on the liquid component.

3. Process according to Claims 1 or 2, characterized in that the solid component is subjected to final sterilization by means of radiation and/or ethylene oxide and the liquid component and the active compound solution are subjected to sterile filtration.

4. Process according to Claims 1 to 3, characterized in that the active compounds are cytostatics, antibiotics, antiseptics or bone growth-promoting substances.

5. Process according to Claim 4, characterized in that methotrexate is used as the active compound.

6. Process according to Claims 1 to 5, characterized in that 2-pyrrolidone, N-methylpyrrolidone, DMSO, tetrahydrofuran, dioxane, ethylene glycol, propanediol or combinations thereof are used as the solvent.

7. Set for the preparation of a bone cement comprising active compounds, composed of separate packs of
(a) a solid component comprising a finely divided polymer of acrylic and/or methacrylic acid esters and if appropriate other additives, such as polymerization catalysts, X-ray contrast media, fillers and dyestuffs, the content of which is about 50 to 75% by weight of the bone cement,
(b) a liquid component comprising an acrylic and/or methacrylic acid ester monomer and if appropriate other additives, such as polymerization accelerators and stabilizers, the content of which is about 25 to 50% by weight of the bone cement, and
(c) a solution of an active compound in an organic solvent, the content of which does not exceed 50% by weight, based on the liquid component,
and if appropriate a device for mixing and/or application of the bone cement.

8. Set according to Claim 7, characterized in that pack unit (a) has been subjected to final sterilization by means of radiation and/or ethylene oxide and the contents of pack units (b) and (c) have been subjected to sterile filtration.

9. Set according to Claim 8, characterized in that pack unit (c) comprises methotrexate as the active compound.

## Revendications

1. Procédé pour la préparation de ciments osseux contenant une substance active, ainsi que de matériaux de substitution osseux obtenus à partir de ceux-ci ou de médicaments retard implantables, le ciment osseux étant composé d'environ 50 à environ 75 % en poids d'un composant solide constitué d'un polymérisat finement divisé d'esters de l'acide acrylique et/ou de l'acide méthacrylique, ainsi qu'éventuellement d'autres additifs tels que catalyseurs de polymérisation, agents de contraste pour rayons X, charges et colorants et d'environ 25 à environ 50 % en poids d'un composant liquide constitué d'un monomère ester de l'acide acrylique et/ou méthacrylique, ainsi qu'éventuellement d'autres additifs tels qu'accélérateurs de polymérisation et stabilisants, lesdits composants étant mélangés pour former une pâte liquide à semi-solide, éventuellement mis sous la forme désirée, puis durcis, caractérisé en ce que l'on dissout la substance active dans un solvant organique dont la proportion ne dépasse pas 50 % en poids, rapporté au composant liquide et en ce que l'on mélange cette solution avec le composant liquide ou solide.

2. Procédé selon la revendication 1, caractérisé en ce que la proportion de solvant est comprise entre 5 et 25 % en poids, de préférence entre 10 et 15 % en poids, rapporté au composant liquide.

3. Procédé selon la revendication 1 ou 2, caractérisé en ce que l'on soumet le composant solide à une stérilisation finale au moyen de rayonnement et/ou de l'oxyde d'éthylène et en ce que l'on filtre, de façon stérile, le composant liquide et la solution de substance active.

4. Procédé selon les revendications 1 à 3, caractérisé en ce que les substances actives sont des cytostatiques, des antibiotiques, des antiseptiques ou des substances favorisant la croissance osseuse.

5. Procédé selon la revendication 4, caractérisé en ce que l'on utilise, comme substance active, le méthotrexate.

6. Procédé selon les revendications 1 à 5, caractérisé en ce qe l'on utilise, comme solvant, la 2-pyrrolidone, la N-méthylpyrrolidone, le DMSO, le tétrahydrofuranne, le dioxane, l'éthlèneglycol, le propanediol ou leurs combinaisons.

7. Set pour la préparation d'un ciment osseux contenant une substance active composé d'emballages séparés
(a) d'un composant solide constitué d'un polymérisat finement divisé d'esters de l'acide acrylique et/ou méthacrylique, ainsi qu'éventuellement d'autres additifs tels que catalyseurs de polymérisation, agents de contraste pour rayons X, charges et colorants, dont la proportion est comprise entre environ 50 et environ 75 % en poids par rapport au ciment osseux,
(b) d'un composant liquide constitué d'un monomère ester de l'acide acrylique et/ou méthacrylique, ainsi qu'éventuellement d'autres additifs tels que des accélérateurs de polymérisation et des stabilisants, dont la proportion est comprise entre environ 25 et environ 50 % en poids par rapport au ciment osseux et
(c) d'une solution d'une substance active dans un solvant organique, dont la proportion ne dépasse pas 50 % en poids, rapporté au composant liquide,
ainsi qu'éventuellement d'un dispositif pour mélanger et/ou produire le ciment osseux.

8. Set selon la revendication 7, caractérisé en ce que l'on soumet l'emballage unitaire (a) à une stérilisation finale au moyen de rayonnement et/ou d'oxyde d'éthylène et le contenu des emballages unitaires (b) et (c) à une filtration stérile.

9. Set selon la revendication 8, caractérisé en ce que du méthotrexate en tant que substance active est contenu dans l'emballage unitaire (c).
